# EUROPEAN PATENT APPLICATION

(11) **EP 2 036 919 A1**
(43) Date of publication of application: **18.03.2009**
(21) Application number: 07767767.2
(22) Date of filing: 28.06.2007
(51) Int. Cl.: C07K 5/062, C07K 1/18, C07K 5/083, C12P 21/02, C12R 1/01, C12R 1/125

(54) **METHOD FOR PURIFICATION OF OLIGOPEPTIDE**

(30) Priority: 28.06.2006 JP 2006177759
(71) Applicant: Kyowa Hakko Bio Co., Ltd., Tokyo 100-8185 (JP)
(72) Inventor: TSUCHIYA, Shizuo, Kyowa-cho, Hofu-shi Yamaguchi 747-8522 (JP); NISHIMURA, Tetsuo, Kyowa-cho, Hofu-shi Yamaguchi 747-8522 (JP); SHINDO, Toshikatsu, Osaka 592-0003 (JP)
(74) Representative: Harding, Charles Thomas
(86) International application number: PCT/JP2007/062973
(87) International publication number: WO 2008/001837

(57) **Abstract**

The present invention provides: a method for purifying an oligopeptide, which comprises a step of contacting a solution comprising the oligopeptide and a neutral amino acid with an ion exchange resin in an effective pH range; the method for purifying an oligopeptide, which comprises (a) a step of passing a solution comprising the oligopeptide and the neutral amino acid through a column packed with an ion exchange resin, and (b) a step of eluting the oligopeptide contacted with the ion exchange resin with an eluting solvent; the above method using a weakly acidic cation exchange resin; the above method using a weakly basic anion exchange resin, etc.

## Description

### Technical Field

The present invention relates to a method for isolating and purifying an oligopeptide from a solution comprising the oligopeptide and a neutral amino acid.

### Background Art

Known methods for producing oligopeptides include, for example, (a) a method for producing them from unprotected L-amino acids using peptide synthetase derived from Bacillus subtilis (see non-patent document No. 1), (b) a method for producing them from L-amino acid amides and L-amino acids using an enzyme having L-amino acid amide hydrolase activity or a substance comprising the enzyme (see patent document No. 1), (c) a method for producing them from L-amino acid esters and L-amino acids using a protein having dipeptide-forming activity (see patent documents Nos. 2 to 4), (d) a method using amino acid ester hydrolase (see patent document No. 5), and (e) a method using an enzyme obtained from a bacterium belonging to the genus Empedobacter (see patent documents Nos. 6 to 9). As an example of the above (a), also known are (f) a method for forming dipeptides which comprises culturing Escherichia coli expressing dipeptide-synthesizing enzyme gene ywfe derived from Bacillus subtilis and alanine dehydrogenase gene ald in a medium containing glucose and ammonium salt in the presence of substrate amino acids (e.g., see patent document No. 10) and the like. Further known examples of the methods include (g) a method by chemical synthesis (see non-patent document No. 2) and a method which is a combination of the methods of the above (a) to (f) and the chemical synthesis method. In these methods, separation of the formed oligopeptides from the remaining impurities (for example, substrates) is necessary. Particularly, the methods using an enzyme [for example, those of the above (a) to (f)] often have problems with separation of the formed oligopeptides from substrates.

Known methods for separating and purifying the formed oligopeptides from substrate amino acids include, for example, methods utilizing various kinds of chromatographies such as gel chromatography, affinity chromatography, chromatography using a synthetic adsorbent resin and high performance liquid chromatography, and a method for purifying the formed oligopeptides by crystallization' (e.g., see non-patent document No. 2). However, the purification methods by chromatography often require sufficient examination of selecting a carrier and a solvent, and it is often difficult to carry out satisfactory purification. Further, synthetic adsorbent resins generally used as a carrier are expensive, and thus are not suitable for large-scale commercial production. The purification method by crystallization also has a problem that when oligopeptides are isolated and purified from a crude product containing a neutral amino acid and when the crude product contains an amino acid which has a low solubility and a good crystallinity, for example, leucine, removing the amino acid from the crude product can be difficult.

A purification method by ion exchange chromatography using relatively inexpensive ion exchange resins is also known (e.g., see non-patent documents Nos. 2 to 4). The above method is effective for the separation and purification of oligopeptides prepared from acidic and basic amino acids as substrates, whose isoelectric points are greatly different from each other, because a strongly acidic cation exchange resin and a strongly basic anion exchange resin can adsorb only the amino acids [for example, a method for isolating glycine which comprises treating an aqueous solution comprising iminodicarboxylic acid and glycine with a weakly basic anion exchange resin to adsorb iminodicarboxylic acid which is an acidic substance onto the resin (see patent document No. 11) is known]. However, separation of oligopeptides composed of neutral amino acids having similar isoelectric points from the neutral amino acids is difficult.

On the other hand, also known are: (1) a method for separating a basic amino acid which comprises treating a neutral aqueous solution comprising several kinds of amino acids with a weakly acidic cation exchange resin to adsorb the basic amino acid onto the resin and eluting it with hydrochloric acid (see patent document No. 12), and (2) a method for isolating and purifying anserine (N-β-alanyl-L-1-methyl-histidine) and carnosine (N-β-alanyl-L-histidine) comprising basic amino acids from a bonito fish soup stock which comprises adsorbing anserine and carnosine onto a weakly acidic cation exchange resin and then eluting them with ammonia (see patent document No. 13). These are the methods that utilize the property of basic amino acids or dipeptides comprising basic amino acids being absorbed onto a weakly acidic cation exchange resin.
Patent document No. 1:
   WO2003/010187 pamphlet
Patent document No. 2:
   W02003/010189 pamphlet
Patent document No. 3:
   WO2003/010307 pamphlet
Patent document No. 4:
   Japanese Published Unexamined Patent Application No. 040034/2005
Patent document No. 5:
   Japanese Published Unexamined Patent Application No. 168405/2005
Patent document No. 6:
   Japanese Published Unexamined Patent Application No. 269905/2005
Patent document No. 7:
   W02004/011652 pamphlet
Patent document No. 8:
   W02004/011653 pamphlet
Patent document No. 9:
   W02004/022733 pamphlet
Patent document No. 10:
   W02006/001379 pamphlet
Patent document No. 11:
   Japanese Published Unexamined Patent Application No. 298366/2005
Patent document No. 12:
   US Patent No. 2549378
Patent document No. 13:
   Japanese Published Examined Patent Application No. 93827/1994
Non-patent document No. 1:
   The June 2005 issue of Fine Chemical, Vol. 34, No. 6, p. 25-35, CMC Publishing Co., Ltd. (2005)
Non-patent document No. 2:
   Nobuo Izumiya, Tetsuo Kato, Haruhiko Aoyagi and Michinori Waki, Fundamentals and Experiments of Peptide Synthesis, Maruzen Co., Ltd. (1985)
Non-patent document No. 3:
   Yoshiharu Izumi, Hachiro Nakagawa and Toshio Miwatani (ed.), Manual for Biochemical Experiments 2, Methods for Separation and Analysis of Proteins, p. 1-20, Kagaku-dojin Publishing Company, Inc. (1985)
Non-patent document No. 4:
   Diaion I, Basic edition, the 18th version, p. 15, Mitsubishi Chemical Corporation, Ion Exchange Resin Div., (November 1st, 2003)

### Disclosure of the Invention

### Problems to be Solved by the Invention

An object of the present invention is to provide a simple method for isolating and purifying an oligopeptide from a solution comprising the oligopeptide and a neutral amino acid.

### Means for Solving the Problems

The present invention relates to the following (1) to (21).
(1) A method for purifying an oligopeptide, which comprises a step of contacting a solution comprising the oligopeptide and a neutral amino acid with an ion exchange resin in an effective pH range.
(2) The method according to (1), which comprises: (a) a step of passing the solution comprising the oligopeptide and the neutral amino acid through a column packed with the ion exchange resin; and (b) a step of eluting the oligopeptide contacted with the ion exchange resin with an eluting solvent.
(3) The method according to (1) or (2), wherein the ion exchange resin is a weakly acidic cation exchange resin.
(4) The method according to (3), wherein the ion exchange resin is a resin having a carboxyl group or a phenolic hydroxy group.
(5) The method according to (3) or (4), wherein the pH of the solution comprising the oligopeptide and the neutral amino acid is in the range of 4 to 14.
(6) The method according to (3) or (4), wherein the pH of the solution comprising the oligopeptide and the neutral amino acid is in the range of 5 to 12.
(7) The method according to (1) or (2), wherein the ion exchange resin is a weakly basic anion exchange resin.
(8) The method according to (7), wherein the ion exchange resin is a resin having a group selected from the group consisting of an amino group, a lower alkylamino group and a di-lower alkylamino group.
(9) The method according to (7), wherein the ion exchange resin is a resin having a group selected from the group consisting of an amino group, a methylamino group and a dimethylamino group.
(10) The method according to any of (7) to (9), wherein the pH of the solution comprising the oligopeptide and the neutral amino acid is in the range of 0 to 9.
(11) The method according to any of (7) to (9), wherein the pH of the solution comprising the oligopeptide and the neutral amino acid is in the range of 1 to 7.
(12) The method according to any of (1) to (11), wherein the neutral amino acid is an amino acid selected from the group consisting of an L-amino acid selected from the group consisting of L-alanine, L-valine, L-leucine, L-isoleucine, L-methionine, L-tryptophan, L-phenylalanine, L-proline, L-serine, L-threonine, L-cysteine, L-tyrosine, L-asparagine and L-glutamine, a D-amino acid which is an optical isomer thereof, a racemic mixture thereof, glycine and β-alanine.
(13) The method according to any of (1) to (11), wherein the neutral amino acid is an amino acid selected from the group consisting of L-alanine, L-valine, L-leucine, L-isoleucine, L-tyrosine, D-alanine, D-valine, D-leucine, D-isoleucine, D-tyrosine, DL-alanine, DL-valine, DL-leucine, DL-isoleucine, DL-tyrosine and glutamine.
(14) The method according to any of (1) to (13), wherein the oligopeptide is a dipeptide or a tripeptide.
(15) The method according to any of (1) to (13), wherein the oligopeptide is a dipeptide.
(16) The method according to (14) or (15), wherein the dipeptide is a dipeptide represented by X-Y (wherein X represents alanine; and Y represents L-valine, L-leucine, L-isoleucine, L-tyrosine, D-valine, D-leucine, D-isoleucine, D-tyrosine, DL-valine, DL-leucine, DL-isoleucine, DL-tyrosine or glutamine).
(17) The method according to any of (1) to (16), wherein the amino acids constituting the oligopeptide are L-amino acids.
(18) The method according to any of (1) to (17), wherein the oligopeptide is an oligopeptide selected from the group consisting of (A) an oligopeptide obtained by a production process which comprises allowing peptide synthetase derived from Bacillus subtilis to act on unprotected L-amino acids, (B) an oligopeptide produced by allowing an enzyme having L-amino acid amide hydrolase activity or a substance containing the enzyme to act on L-amino acid amides and L-amino acids, (C) an oligopeptide produced by allowing a protein having the activity to form a dipeptide to act on L-amino acid esters and L-amino acids, (D) an oligopeptide produced by the action of amino acid ester hydrolase, (E) an oligopeptide produced by the action of an enzyme obtained from a bacterium belonging to the genus Empedobacter, and (F) an oligopeptide obtained by chemical synthesis.
(19) A process for producing an oligopeptide, which comprises a step of utilizing the method described in any of (1) to (18).
(20) An oligopeptide produced by the process described in (19).
(21) The oligopeptide according to (20), which does not contain an amino acid as an impurity.

### Effect of the Invention

The present invention provides a simple method for isolating and purifying am oligopeptide from a solution comprising the oligopeptide and a neutral amino acid.

### Best Modes for Carrying Out the Invention

There is no specific restriction as to the neutral amino acid of the present invention so long as it is an amino acid giving a pH around neutrality when dissolved in water. Examples of the neutral amino acids are L-amino acids selected from the group consisting of L-alanine, L-valine, L-leucine, L-isoleucine, L-methionine, L-tryptophan, L-phenylalanine, L-proline, L-serine, L-threonine, L-cysteine, L-tyrosine, L-asparagine and L-glutamine, D-amino acids which are optical isomers thereof, racemic mixtures thereof, glycine, β-alanine, γ-aminobutyric acid, carnitine and the like.

The oligopeptides to which the purification method of the present invention is applicable include, for example, oligopeptides in which 2 to 10 amino acids are linked in the form of a straight chain or a ring, specifically, dipeptides, tripeptides, tetrapeptides, pentapeptides, hexapeptides, heptapeptides, octapeptides, nonapeptides, decapeptides, cyclotetrapeptides, cyclopentapeptides, cyclohexapeptides, cycloheptapeptides, cyclooctapeptides, cyclononapeptides, cyclodecapeptides and the like. Preferred are dipeptides and tripeptides consisting of 2 to 3 amino acids.

The kind of amino acids constituting oligopeptides to which the purification method of the present invention is applicable is not specifically limited, and examples of the amino acids include naturally occurring L-amino acids, D-amino acids which are isomers thereof, racemic mixtures thereof, glycine, β-alanine, γ-aminobutyric acid, carnitine and the like. It is preferred that oligopeptides contain one or more neutral amino acids, more preferably one or more amino acids which are neutral L-amino acids, D-amino acids which are isomers thereof, racemic mixtures thereof, glycine, β-alanine and the like. The constitutive amino acids may be the same or different. Specifically, it is preferred that oligopeptides contain, for example, one or more amino acids which are L-amino acids selected from the group consisting of L-alanine, L-valine, L-leucine, L-isoleucine, L-methionine, L-tryptophan, L-phenylalanine, L-proline, L-serine, L-threonine, L-cysteine, L-tyrosine, L-asparagine and L-glutamine, D-amino acids which are isomers thereof, racemic mixtures thereof, glycine, β-alanine and the like.

Preferred oligopeptides of the present invention are those containing preferred neutral amino acids mentioned above in which are same or different and in number of 1 to 10. More preferred are dipeptides and tripeptides which contain one or more neutral amino acids which are same or different. More specifically, preferred examples of the oligopeptides are: a tripeptide represented by formula (1) Xa-YY-ZZ (wherein Xa represents an L-amino acid selected from the group consisting of L-alanine, L-valine, L-leucine, L-isoleucine, L-methionine, L-tryptophan, L-phenylalanine, L-proline, L-serine, L-threonine, L-cysteine, L-tyrosine, L-asparagine and L-glutamine, a D-amino acid which is an isomer thereof, a racemic mixture thereof, glycine or β-alanine; and YY and ZZ, which may be the same or different, each represent a naturally occurring L-amino acid, a D-amino acid which is an isomer thereof, a racemic mixture thereof, glycine, β-alanine, γ-aminobutyric acid, carnitine or the like); a dipeptide represented by formula (2) Xa-YY (wherein-Xa and YY have the same meanings as defined above, respectively); a tripeptide represented by formula (3) Xa-Ya-ZZ (wherein Xa and ZZ have the same meanings as defined above, respectively; and Ya represents an L-amino acid selected from the group consisting of L-alanine, L-valine, L-leucine, L-isoleucine, L-methionine, L-tryptophan, L-phenylalanine, L-proline, L-serine, L-threonine, L-cysteine, L-tyrosine, L-asparagine and L-glutamine, a D-amino acid which is an isomer thereof, a racemic mixture thereof, glycine or β-alanine); a dipeptide represented by formula (4) Xa-Ya (wherein Xa and Ya have the same meanings as defined above, respectively); a tripeptide represented by formula (5) Xa-Ya-Za (wherein Xa and Ya have the same meanings as defined above, respectively; and Za represents an L-amino acid selected from the group consisting of L-alanine, L-valine, L-leucine, L-isoleucine, L-methionine, L-tryptophan, L-phenylalanine, L-proline, L-serine, L-threonine, L-cysteine, L-tyrosine, L-asparagine and L-glutamine, a D-amino acid which is an isomer thereof, a racemic mixture thereof, glycine or β-alanine); formula (6) Xa-YY-Za (wherein Xa, YY and Za have the same meanings as defined above, respectively); a tripeptide represented by formula (7) XX-Ya-Za (wherein XX represents a naturally occurring L-amino acid, a D-amino acid which is an isomer thereof, a racemic mixture thereof, glycine, β-alanine, γ-aminobutyric acid, carnitine or the like; and Ya and Za have the same meanings as defined above, respectively); and a dipeptide represented by formula (8) XX-Ya (wherein XX and Ya have the same meanings as defined above, respectively); more preferred examples are: a tripeptide represented by formula (9) Xb-YY-ZZ (wherein Xb represents an L-amino acid selected from the group consisting of L-alanine, L-methionine, L-serine and L-threonine, a D-amino acid which is an isomer thereof, a racemic mixture thereof, glycine or β-alanine; and YY and ZZ have the same meanings as defined above, respectively); a dipeptide represented by formula (10) Xb-YY (wherein Xb and YY have the same meanings as defined above, respectively); a tripeptide represented by formula (11) Xb-Ya-ZZ (wherein Xb, Ya and ZZ have the same meanings as defined above, respectively); a dipeptide represented by formula (12) Xb-Ya (wherein Xb and Ya have the same meanings as defined above, respectively); a tripeptide represented by formula (13) Xb-YY-Za (wherein Xb, YY and Za have the same meanings as defined above, respectively); and a tripeptide represented by formula (14) Xb-Ya-Za (wherein Xb, Ya and Za have the same meanings as defined above, respectively); further preferred examples are: a tripeptide represented by formula (15) Xc-YY-ZZ (wherein Xc represents L-alanine; and YY and ZZ have the same meanings as defined above, respectively); a dipeptide represented by formula (16) Xc-YY (wherein Xc and YY have the same meanings as defined above, respectively); a tripeptide represented by formula (17) Xc-Ya-ZZ (wherein Xc, Ya and ZZ have the same meanings as defined above, respectively); a dipeptide represented by formula (18) Xc-Ya (wherein Xc and Ya have the same meanings as defined above, respectively); a tripeptide represented by formula (19) Xc-YY-Za (wherein Xc, YY and Za have the same meanings as defined above, respectively); and a tripeptide represented by formula (20) Xc-Ya-Za (wherein Xc, Ya and Za have the same meanings as defined above, respectively); and further, more preferred examples are: a tripeptide represented by formula (21) Xc-Yb-ZZ (wherein Xc and ZZ have the same meanings as defined above, respectively; and Yb, which may be the same or different, represents an L-amino acid selected from the group consisting of L-valine, L-leucine, L-isoleucine, L-tyrosine and L-glutamine, a D-amino acid which is an isomer thereof, a racemic mixture thereof, glycine or β-alanine); a dipeptide represented by formula (22) Xc-Yb (wherein Xc and Yb have the same meanings as defined above, respectively); a dipeptide represented by formula (23) Xc-Yc (wherein Xc has the same meaning as defined above; and Yc represents L-valine, L-leucine, L-isoleucine, L-tyrosine, D-valine, D-leucine, D-isoleucine, D-tyrosine, DL-valine, DL-leucine, DL-isoleucine, DL-tyrosine or glutamine); a tripeptide represented by formula (24) Xc-YY-Zb (wherein Xc and YY have the same meanings as defined above, respectively; and Zb, which may be the same or different, represents an L-amino acid selected from the group consisting of L-valine, L-leucine, L-isoleucine, L-tyrosine and L-glutamine, a D-amino acid which is an isomer thereof, a racemic mixture thereof, glycine or β-alanine); a tripeptide represented by formula (25) Xc-Yb-Za (wherein Xc, Yb and Za have the same meanings as defined above, respectively); or formula (26) Xc-Yb-Zb (wherein Xc, Yb and Zb have the same meanings as defined above, respectively).

Of the oligopeptides represented by the above formulae (1) to (26), preferred are those consisting of amino acids which are all L-amino acids. More specifically, preferred examples of the oligopeptides are alanylvaline, alanylleucine, alanylisoleucine, alanyltyrosine, alanylglutamine and the like, and more preferred examples are L-alanyl-L-valine, L-alanyl-L-leucine, L-alanyl-L-isoleucine, L-alanyl-L-tyrosine, L-alanyl-L-glutamine and the like.

The solution comprising an oligopeptide and a neutral amino acid used in the present invention is a solution comprising the above oligopeptide and one or more neutral amino acids described above. The solution may further comprise ionic substances such as other amino acids, proteins and inorganic salts, and nonionic substances such as sugars and pigments. However, in order to carry out the purification method of the present invention, it is preferred that the amount of the ionic substances contained in the solution is small. The solution is preferably an aqueous solution or an aqueous alcoholic solution containing methanol, ethanol, propanol, 2-propanol or the like, and an aqueous solution is more preferred.

The solution comprising an oligopeptide and a neutral amino acid can be obtained by: obtaining an enzymatic reaction solution or a culture, for example, by the above-mentioned (a) method for producing an oligopeptide from unprotected L-amino acids using peptide synthetase derived from Bacillus subtilis, (b) method for producing an oligopeptide from L-amino acid amides and L-amino acids using an enzyme having L-amino acid amide hydrolase activity or a substance comprising the enzyme, (c) method for producing an oligopeptide from L-amino acid esters and L-amino acids using a protein having dipeptide-forming activity, (d) method for producing an oligopeptide using amino acid ester hydrolase, (e) method for producing an oligopeptide using an enzyme obtained from a bacterium belonging to the genus Empedobacter, or (f) method for forming a dipeptide which comprises culturing Escherichia coli expressing dipeptide-synthesizing enzyme gene ywfe derived from Bacillus subtilis and alanine dehydrogenase gene ald in a medium containing glucose and ammonium salt in the presence of substrate amino acids; subjecting the obtained enzymatic reaction solution or the culture to centrifugation, membrane separation or filtration to remove cells; and then desalting the obtained solution by electrodialysis or by using a strongly acidic cation exchange resin or the like. The solution can also be obtained by dissolving a crude product obtained by (g) a method for producing an oligopeptide by chemical synthesis, a method for producing an oligopeptide which is a combination of the methods of the above (a) to (f) and the chemical synthesis method, or the like in water or a mixed solvent of alcohol such as methanol, ethanol, propanol or 2-propanol and water.

The ion exchange resin used in the present invention is not specifically limited, and various kinds of ion exchange resins can be appropriately used. Preferred are weakly acidic cation exchange resins and weakly basic anion exchange resins. Examples of the weakly acidic cation exchange resins include resins having a carboxyl group, a phenolic hydroxy group or the like as a functional group thereon, more specifically acrylic or methacrylic resins such as Diaion WK-40 (Mitsubishi Chemical Corporation), MAC3 (Dow Chemical Company), CNP80ws and CNPLF (Bayer AG), and IRC50 and IRC76 (Amberlite) and resins prepared therefrom. As the ionic form of these weakly acidic cation exchange resins, H-form is preferred. Examples of the weakly basic anion exchange resins include resins having a primary amino group, a secondary amino group or a tertiary amino group as a functional group thereon, specifically resins having an amino group, a lower alkylamino group, a di-lower alkylamino group or the like, more specifically resins having an amino group, a methylamino group, an ethylamino group, a dimethylamino group, a diethylamino group or the like, further specifically, acrylic or styrene reins such as Diaion WA-10, 21 and 30 (Mitsubishi Chemical Corporation) and resins prepared therefrom. As the ionic form of these weakly basic anion exchange resins, Cl-form is preferred. The purification method of oligopeptides of the present invention is carried out ordinarily by using one kind of ion exchange resin among these resins, but may also be carried out by using appropriately combined 2 to 4 kinds of ion exchange resins selected from a strongly acidic cation exchange resin, a strongly basic anion exchange resin and the like in addition to the above weakly acidic cation exchange resin and weakly basic anion exchange resin, if necessary. Examples of the combinations include those of 2 to 4 kinds of ion exchange resins respectively having different functional groups thereon; for instance, a combination of a strongly acidic cation exchange resin, a strongly basic anion exchange resin, a weakly acidic cation exchange resin and a weakly basic anion exchange resin; a combination of a strongly acidic cation exchange resin, a weakly acidic cation exchange resin and a weakly basic anion exchange resin; a combination of a strongly basic anion exchange resin, a weakly acidic cation exchange resin and a weakly basic anion exchange resin; a combination of a strongly acidic cation exchange resin and a weakly acidic cation exchange resin; a combination of a strongly basic anion exchange resin and a weakly acidic cation exchange resin; a combination of a strongly acidic cation exchange resin and a weakly basic anion exchange resin; a combination of a strongly basic anion exchange resin and a weakly basic anion exchange resin; and a combination of a weakly acidic cation exchange resin and a weakly basic anion exchange resin.

The step of contacting a solution comprising an oligopeptide and a neutral amino acid with an ion exchange resin in an effective pH range of the present invention can be carried out, for example, by passing the solution comprising the oligopeptide and the neutral amino acid through a column packed with an ion exchange resin in an effective pH range, by adding the solution comprising the oligopeptide and the neutral amino acid to an aqueous solution containing an ion exchange resin dispersed therein, followed by mixing in an effective pH range, or by adding an ion exchange resin to the solution comprising the oligopeptide and the neutral amino acid, followed by mixing them in an effective pH range.' It is preferred to carry out the step by passing the solution comprising the oligopeptide and the neutral amino acid through a column packed with an ion exchange resin in an effective pH range.

When a solution comprising an oligopeptide and a neutral amino acid is passed through a column packed with an ion exchange resin, it is preferred that the concentrations of the oligopeptide and the neutral amino acid contained in the solution is low, and water may be added to the solution, if necessary.
The "effective pH range" refers to a pH range appropriate for utilizing an ion exchange resin, and an appropriate pH range is determined according to the kind of an ion exchange resin used. For example, when a weakly acidic cation exchange resin is used, the pH of the' solution is in the range of 4 to 14, preferably 5 to 14, more preferably 5 to 12, further preferably 6 to 12, and further more preferably 7 to 10. When a weakly basic anion exchange resin is used, the pH of the solution is in the range of 0 to 9, preferably 1 to 9, more preferably 1 to 7, further preferably 2 to 7, and further more preferably 2 to 6.

The pH of the solution comprising an oligopeptide and a neutral amino acid used should be adjusted to an optimum pH (effective pH range) according to the kind of the ion exchange resin used. For example, when a weakly acidic cation exchange resin is used, the pH of the solution is in the range of 4 to 14, preferably 5 to 14, more preferably 5 to 12, further preferably 6 to 12, and further more preferably 7 to 10. The solution is preferably in a basic state. When a weakly basic anion exchange resin is used, the pH of the solution is in the range of 0 to 9, preferably 1 to 9, more preferably 1 to 7, further preferably 2 to 7, and further more preferably 2 to 6. The solution is preferably in an acidic state. Accordingly, it is preferred to adjust the pH of the solution to the above preferable range using acids such as hydrochloric acid, sulfuric acid, acetic acid and malic acid, and bases such as sodium hydroxide, sodium carbonate, sodium hydrogencarbonate and aqueous ammonia.

In the present invention, the ion exchange resin is used preferably in such an amount that the amount of functional groups (ion exchange groups) on the ion exchange resin sufficiently exceeds the total amount of ions in the solution comprising an oligopeptide and a neutral amino acid. For example, when a weakly acidic cation exchange resin is used, the ion exchange resin is preferably used in such an amount that the amount of ion exchange groups exceeds the total amount of cation in the solution, and when a weakly basic anion exchange resin is used, the ion exchange resin is preferably used in such an amount that the amount of ion exchange groups exceeds the total amount of anion in the solution.

As the column which is used when a solution comprising an oligopeptide and a neutral amino acid is passed through a column packed with an ion exchange resin in the purification method of the present invention, any columns used for the purification of chemical substances may be used. It is preferred to select a column so that the ratio of height of resin layer/inner diameter of a column becomes larger when an ion exchange resin is packed in the column, and it is more preferred to select a column so that the ratio of height of resin layer/inner diameter of a column will be 3.5 or more.

In order to pass a solution comprising an oligopeptide and a neutral amino acid used in the purification method of the present invention through a column packed with an ion exchange resin, the solution may be passed, for example, either from the upper part of the column packed with an ion exchange resin, the so-called upper layer of the column bed, or from the lower part of the column, the so-called lower layer of the column bed. It is more preferred to pass the solution through the column from the upper layer of the column bed. As to the passing speed, the solution is passed preferably at a space velocity of 2 (1/hour) or less, more preferably 1 (1/hour) or less.

There is no specific restriction as to the eluting solvent used for elution after a solution comprising an oligopeptide and a neutral amino acid is passed through a column packed with an ion exchange resin. Preferred examples of the solvents include water (either deionized or not), acidic aqueous solutions having a concentration of 0.02 to 6 mol/L (e.g., aqueous solutions of hydrochloric acid, sulfuric acid, acetic acid and malic acid), basic aqueous solutions having a concentration of 0.02 to 6 mol/L (e.g., aqueous solutions of sodium hydroxide, sodium carbonate, sodium hydrogencarbonate and ammonia), and a solvent having the same composition as the solvent of the solution comprising an oligopeptide and a neutral amino acid. Particularly, when a weakly acidic cation exchange resin is used, acidic aqueous solutions having a concentration of 0.02 to 6 mol/L (e.g., aqueous solutions of hydrochloric acid, sulfuric acid, acetic acid and malic acid) are preferred, and when a weakly basic anion exchange resin is used, basic aqueous solutions having a concentration of 0.02 to 6 mol/L (e.g., aqueous solutions of sodium hydroxide, sodium carbonate, sodium hydrogencarbonate and ammonia) are preferred.

According to the purification method of the present invention, an oligopeptide can be isolated and purified, for example, by passing a solution comprising the oligopeptide and a neutral amino acid through a column packed with an ion exchange resin, and passing the above eluant, preferably continuously, through the column to contact the oligopeptide with the ion exchange resin and to elute the oligopeptide. This method is particularly effective for purification of an oligopeptide composed of neutral amino acids, and can remove the neutral amino acid from the solution comprising the oligopeptide and the neutral amino acid to purify the oligopeptide. As to the passing speed of the eluant, the eluant is passed preferably at a space velocity of 0.3 to 10 (1/hour), more preferably 0.5 to 2 (1/hour).

As described above, the purification method of the present invention can effectively separate an oligopeptide from an amino acid, a salt, a metal ion and the like in a solution comprising the oligopeptide and a neutral amino acid, and is particularly effective for separation of an oligopeptide containing a neutral amino acid among the constituent amino acids, preferably an oligopeptide composed of neutral amino acids and the neutral amino acid.
In addition to the above procedure utilizing a column, the purification method of the present invention can also be carried out by diluting a mixture of a solution comprising an oligopeptide and a neutral amino acid and an ion exchange resin with water or the like , if necessary, and after mixing, separating the ion exchange resin by means of filtration or the like.

The purification method of the present invention can also be easily applied to a solution comprising an amino acid having a good crystallinity such as L-valine, L-leucine, L-isoleucine, L-tyrosine or L-glutamine. An oligopeptide having a high purity can be obtained from the above eluate or filtrate obtained by the purification method of the present invention by using known methods usually performed for producing peptides, for example, operations such as desalting, concentration and crystallization.

As described above, the purification method of the present invention can be included as one of the steps in a process for producing an oligopeptide comprising a neutral amino acid as a constitutive amino acid. The oligopeptide produced by a production process which comprises the purification method of the present invention as one of the steps is characterized by a remarkably small content of impurities such as a neutral amino acid.
Hereinafter, the present invention will be described more specifically with reference to Examples. However, the scope of the present invention is not limited to these Examples.

### Example 1

### Purification of Ala-Ile

An aqueous solution (1000 ml) comprising Ala-Ile (2.75 g), Ala (2.50 g), Ile (0.40 g) and Ala-Ala (1.89 g) was passed through a column packed with WK-40 (1000 ml, Mitsubishi Chemical Corporation) from the upper layer of the column bed at a space velocity of 0.5 (1/hour), and then water was continuously passed from the upper layer of the column bed. The separation process was observed by carrying out monitoring with an UV sensor equipped at the outlet of the column. Then, fractions in which Ala-Ile was eluted were collected and the components were analyzed, whereby it was revealed that Ala-Ile was contained at a yield of 83.1%. The removal rates of Ile and Ala were 98.1% and 100.0%, respectively.

### Example 2

### Purification of Ala-Leu

An aqueous solution (1000 ml) comprising Ala-Leu (10.0 g), Ala (0.80 g), Leu (0.99 g) and Ala-Ala (1.49 g) was passed through a column packed with WK-40 (720 ml, Mitsubishi Chemical Corporation) from the upper layer of the column bed at a space velocity of 0.5 (1/hour), and then 0.02 mol/L hydrochloric acid was continuously passed from the upper layer of the column bed. The separation process was observed by carrying out monitoring with an UV sensor equipped at the outlet of the column. Then, fractions in which Ala-Leu was eluted were collected and the components were analyzed, whereby it was revealed that Ala-Leu was contained at a yield of 85.9%. The removal rates of Leu and Ala were 99.0% and 99.9%, respectively.

### Example 3

### Purification of Ala-Val

An aqueous solution (800 ml) comprising Ala-Val (7.84 g), Ala (0.53 g), Val (1.49) and Ala-Ala (1.27 g) was passed through a column packed with WK-40 (1000 ml, Mitsubishi Chemical Corporation) from the upper layer of the column bed at a space velocity of 0.5 (1/hour), and then water was continuously passed from the upper layer of the column bed. The separation process was observed by carrying out monitoring with an UV sensor equipped at the outlet of the column. Then, fractions in which Ala-Val was eluted were collected and the components were analyzed, whereby it was revealed that Ala-Val was contained at a yield of 86.0%. The removal rates of Val and Ala were 95.0% and 96.7%, respectively.

### Example 4

### Purification of Ala-Tyr

An aqueous solution (800 ml) comprising Ala-Tyr (7.88 g), Ala (0.57 g), Tyr (0.21) and Ala-Ala (1.30 g) was passed through a column packed with WK-40 (1000 ml, Mitsubishi Chemical Corporation) from the upper layer of the column bed at a space velocity of 0.5 (1/hour), and then water was continuously passed from the upper layer of the column bed. The separation process was observed by carrying out monitoring with an UV sensor equipped at the outlet of the column. Then, fractions in which Ala-Tyr was eluted were collected and the components were analyzed, whereby it was revealed that Ala-Tyr was contained at a yield of 84.6%. The removal rates of Tyr and Ala were 96.2% and 97.7%, respectively.

### Example 5

### Purification of Ala-Val

An aqueous solution (500 ml) comprising Ala-Val (5.32 g), Ala (0.86 g), Val (1.18) and Ala-Ala (3.73 g) was passed through a column packed with WA-21 (1000 ml, Mitsubishi Chemical Corporation) from the upper layer of the column bed at a space velocity of 0.5 (1/hour), and then water was continuously passed from the upper layer of the column bed. The separation process was observed by carrying out monitoring with an UV sensor equipped at the outlet of the column. Then, fractions in which Alp-Val was eluted were collected and the components were analyzed, whereby it was revealed that Ala-Val was contained at a yield of 74.5%. The removal rates of Val and Ala were 100.0% and 100.0%, respectively.

### Example 6

### Production Process of Ala-Leu

Sulfuric acid was added to a culture (4 L) comprising Ala-Leu (44.7 g/l), impurities such as Ala and Leu, and'cells of Eschelichia coli in which a dipeptide synthetase derived from Bacillus subtilis was expressed, which was obtained according to the method described in W02004/058960, to adjust pH to 3.0, and the resulting mixture was centrifuged to sediment the cells. The supernatant obtained by sedimenting the cells was passed through a column packed with a strongly acidic cation exchange resin SK-1B(H⁺)(9 L, Mitsubishi Chemical Corporation) from the upper layer of the column bed at a space velocity of 1.0 (1/hour), and then water was continuously passed from the upper layer of the column bed to adsorb Ala-Leu onto the resin. Further, 0.7 mol/L sodium hydroxide was passed through the column from the upper layer of the column bed to elute Ala-Leu adsorbed onto the resin. Among the eluates, fractions containing Ala-Leu were collected. The obtained fractions contained 92% of Ala-Leu in the above culture. However, no change was observed in the ratio of impurities such as Ala and Leu contained therein to Ala-Leu. This fraction was passed through a column packed with WK-40(H⁺) (12 L, Mitsubishi Chemical Corporation) from the upper layer of the column bed at a space velocity of 0.5 (1/hour), and then water was continuously passed from the upper layer of the column bed. Monitoring was carried out with an UV sensor equipped at the outlet of the column and a fraction containing Ala-Leu was obtained. By this operation, Leu and Ala as impurities were removed by 98.3% and 99.4%, respectively. The fraction was crystallized, whereby 98.7 g of Ala-Leu was obtained.

### Industrial Applicability

The method for purifying an oligopeptide provided'by the present invention, which comprises the step of contacting a solution comprising the oligopeptide and a neutral amino acid with an ion exchange resin in an effective pH range, is useful as the method for isolating and purifying the oligopeptide.

## Claims

1. A method for purifying an oligopeptide, which comprises a step of contacting a solution comprising the oligopeptide and a neutral amino acid with an ion exchange resin in an effective pH range.

2. The method according to Claim 1, which comprises: (a) a step of passing the solution comprising the oligopeptide and the neutral amino acid through a column packed with the ion exchange resin; and (b) a step of eluting the oligopeptide contacted with the ion exchange resin with an eluting solvent.

3. The method according to Claim 1 or 2, wherein the ion exchange resin is a weakly acidic cation exchange resin.

4. The method according to Claim 3, wherein the ion exchange resin is a resin having a carboxyl group or a phenolic hydroxy group.

5. The method according to Claim 3 or 4, wherein the pH of the solution comprising the oligopeptide and the neutral amino acid is in the range of 4 to 14.

6. The method according to Claim 3 or 4, wherein the pH of the solution comprising the oligopeptide and the neutral amino acid is in the range of 5 to 12.

7. The method according to Claim 1 or 2, wherein the ion exchange resin is a weakly basic anion exchange resin.

8. The method according to Claim 7, wherein the ion exchange resin is a resin having a group selected from the group consisting of an amino group, a lower alkylamino group and a di-lower alkylamino group.

9. The method according to Claim 7, wherein the ion exchange resin is a resin having a group selected from the group consisting of an amino group, a methylamino group and a dimethylamino group.

10. The method according to any of Claims 7 to 9, wherein the pH of the solution comprising the oligopeptide and the neutral amino acid is in the range of 0 to 9.

11. The method according to any of Claims 7 to 9, wherein the pH of the solution comprising the oligopeptide and the neutral amino acid is in the range of 1 to 7.

12. The method according to any of Claims 1 to 11, wherein the neutral amino acid is an amino acid selected from the group consisting of an L-amino acid selected from the group consisting of L-alanine, L-valine, L-leucine, L-isoleucine, L-methionine, L-tryptophan, L-phenylalanine, L-proline, L-serine, L-threonine, L-cysteine, L-tyrosine, L-asparagine and L-glutamine, a D-amino acid which is an optical isomer thereof, a racemic mixture thereof, glycine and β-alanine.

13. The method according to any of Claims 1 to 11, wherein the neutral amino acid is an amino acid selected from the group consisting of L-alanine, L-valine, L-leucine, L-isoleucine, L-tyrosine, D-alanine, D-valine, D-leucine, D-isoleucine, D-tyrosine, DL-alanine, DL-valine, DL-leucine, DL-isoleucine, DL-tyrosine and glutamine.

14. The method according to any of Claims 1 to 13, wherein the oligopeptide is a dipeptide or a tripeptide.

15. The method according to any of Claims 1 to 13, wherein the oligopeptide is a dipeptide.

16. The method according to Claim 14 or 15, wherein the dipeptide is a dipeptide represented by X-Y (wherein X represents alanine; and Y represents L-valine, L-leucine, L-isoleucine, L-tyrosine, D-valine, D-leucine, D-isoleucine, D-tyrosine, DL-valine, DL-leucine, DL-isoleucine, DL-tyrosine or glutamine).

17. The method according to any of Claims 1 to 16, wherein the amino acids constituting the oligopeptide are L-amino acids.

18. The method according to any of Claims 1 to 17, wherein the oligopeptide is an oligopeptide selected from the group consisting of (A) an oligopeptide obtained by a production process which comprises allowing peptide synthetase derived from Bacillus subtilis to act on unprotected L-amino acids, (B) an oligopeptide produced by allowing an enzyme having L-amino acid amide hydrolase activity or a substance containing the enzyme to act on L-amino acid amides and L-amino acids, (C) an oligopeptide produced by allowing a protein having the activity to form a dipeptide to act on L-amino acid esters and L-amino acids, (D) an oligopeptide produced by the action of amino acid ester hydrolase, (E) an oligopeptide produced by the action of an enzyme obtained from a bacterium belonging to the genus Empedobacter, and (F) an oligopeptide obtained by chemical synthesis.

19. A process for producing an oligopeptide, which comprises a step of utilizing the method described in any of Claims 1 to 18.

20. An oligopeptide produced by the process described in Claim 19.

21. The oligopeptide according to Claim 20, which does not contain an amino acid as an impurity.
